# EUROPÄISCHE PATENTSCHRIFT

(11) **EP 0 900 773 B1**
(45) Veröffentlichungstag und Bekanntmachung des Hinweises auf die Patenterteilung: **07.11.2001**
(21) Anmeldenummer: 98115243.2
(22) Anmeldetag: 13.08.1998
(51) Int. Cl.: C07C 5/25

(54) **Verfahren zur Herstellung von C4-C6-Alkenen mit innenständiger Doppelbindung**
Process for the preparation of C4-C6 alkenes with terminal double bound
Procédé pour la préparation d'alcènes à double liaison en bout de chaine

(30) Priorität: 26.08.1997 DE 19737019
(43) Veröffentlichungstag der Anmeldung: 10.03.1999
(73) Patentinhaber: EC ERDÖLCHEMIE GMBH, D-50769 Köln (DE)
(72) Erfinder: Stüwe, Arnd, Dr., 51373 Leverkusen (DE); Michel, Jörg-Uwe, 41539 Dormagen (DE); Baum, Matthias, Dr., 41542 Dormagen (DE); Kaledat, Franz, 41466 Neuss (DE)
(74) Vertreter: Preece, Michael

(56) Entgegenhaltungen:
- US-A- 4 132 745
- US-A- 4 849 576

## Beschreibung

Die vorliegende Erfindung betrifft ein Verfahren zur Herstellung von C₄-C₆-Alkenen mit innenständiger Doppelbindung durch Hydroisomerisierung von C₄-C₆-Alkenen mit endständiger Doppelbindung, in welchem der vom Reaktor ablaufende Produktstrom in einen Aufarbeitungsstrom und einen Rückführstrom aufgeteilt wird und der Rückführstrom an den Reaktoreingang zurückgeführt wird, wo er gemeinsam mit den C₄-C₆-Alkenen mit endständiger Doppelbindung und dem H₂ das Feed für den Reaktor bildet. Das Verfahren dient vor allem der Umwandlung von Buten-1 in Buten-2.

Alkene mit innenständiger Doppelbindung, beispielsweise Buten-2, sind begehrte Alkylierungsmittel zur Alkylierung von n-Alkanen und/oder i-Alkanen, wobei sich wertvolle Motorkraftstoffe ("Alkylatbenzin") ergeben. Solche Alkylate aus Olefinen mit innenständiger Doppelbindung ergeben im Treibstoffsektor bessere Eigenschaften als Alkylate von Olefinen mit endständiger Doppelbindung. So ergibt beispielsweise n-Buten-1 mit n-Butan/i-Butan ein Alkylatbenzin mit einer Research Octanzahl ROZ von 92,5, während das entsprechende Alkylat von Buten-2 eine ROZ von 98,5 aufweist. Die Verschiebung endständiger Doppelbindungen in eine innenständige Position des Olefins stellt daher einen wichtigen Schritt zur Gewinnung von hochoctanigem Benzin dar.

Die Umwandlung beispielsweise von Buten-1 in Buten-2 wird seit langem als sogenannte Hydroisomerisierung in Gegenwart von Wasserstoff an einem für die Hydrierung geeigneten Katalysator durchgeführt. So ist aus US 3.531.545 bekannt, hierzu einen Katalysator mit einem Gehalt an Pd oder Pt auf SiO₂ oder Al₂O₃ als Träger einzusetzen, der jedoch zur Vermeidung der unerwünschten Überhydrierung mit Hilfe von Schwefelverbindungen in seiner Aktivität gedämpft wird. Als Folge hiervon muß jedoch die Hydroisomerisierung bei einer Temperatur von bis zu 155°C durchgeführt werden. Das thermodynamische Gleichgewicht liegt jedoch bei höheren Temperaturen mehr auf der Seite des Buten-1, so daß bei höheren Temperaturen geringere Hydroisomerisierungsausbeuten erzielt werden können. Gemäß FR 2.438.084 kann eine Selektivitätssteigerung eines Pd/Al₂O₃-Katalysators neben einer Behandlung mit isomerisierungsausbeuten erzielt werden können. Gemäß FR 2.438.084 kann eine Selektivitätssteigerung eines Pd/Al₂O₃-Katalysators neben einer Behandlung mit Schwefelverbindungen auch durch eine Behandlung mit Ammoniak oder Kohlenmonoxid herbeigeführt werden. Eine weitere Variante der Selektivitätssteigerung wird in US 4.132.745 beschrieben, worin ein Pd/Al₂O₃-Katalysator mit Schwefelwasserstoff und anschließend mit Wasserstoff behandelt wird; bei 80 bis 100°C wird mit einem solchen Katalysator eine Hydroisomerisierung durchgeführt, bei der begleitendes Butadien weitgehend durch Hydrierung eliminiert wird, aber auch ein beträchtlicher Teil von Buten in unerwünschter Weise zum gesättigten Butan hydriert wird. DE-A 31 40 573 beschreibt eine Hydroisomerisierung von Buten-1 zu Buten-2 an einem Katalysator mit 0,3 % Pd auf hochreinem Al₂O₃, die im Anschluß an die Oligomerisierung von i-Buten und in Gegenwart des Oligomerisats vorgenommen wird. Die Isomerisierung wird bei 120°C und einem molaren Verhältnis von Wasserstoff zu Kohlenwasserstoffen von 0,5 durchgeführt. Aus EP-A 338 309 ist bekannt, daß Alkene mit endständiger Doppelbindung zu Alkenen mit innenständiger Doppelbindung isomerisiert werden können, wenn als Katalysator ein makroporöser oder gelförmiger Kationenaustauscher in der H+-Form eingesetzt wird, der 0,001 bis 10 eines Metalls der VIII. Nebengruppe des Periodensystems der Elemente (Mendelejew) pro Liter Kationenaustauscher enthält.

Es wurde nun gefunden, daß der Isomerisierungsgrad von C₄-C₆-Alkenen mit endständiger Doppelbindung zu C₄-C₆-Alkenen mit innenständiger Doppelbindung in einer nicht erwarteten Weise um einen beträchtlichen Betrag in Richtung des thermodynamischen Gleichgewichts vergrößert wird, wenn man den vom Reaktor ablaufenden Produktstrom in einen Aufarbeitungsstrom und einen Rückführstrom aufteilt und den Rückführstrom in das Feed oder an den Reaktoreingang zurückführt. Dieser zurückgeführte Teilstrom kann gegebenenfalls gekühlt werden. Die Zurückführung bringt zunächst einen erhöhten Massenstrom durch den Hydroisomerisierungsreaktor und eine Verdünnung der zu isomerisierenden C₄-C₆-Alkene mit endständiger Doppelbindung. Diese Merkmale bedingen eine Verringerung der Raum-Zeit-Ausbeute und müßten daher vom Fachmann grundsätzlich negativ bewertet werden. Die Vergrößerung des Massenstroms geht jedoch gleichzeitig einher mit einer besseren Löslichkeit des eingesetzten Wasserstoffs und läßt auch die Möglichkeit einer Temperaturabsenkung zu. Diese zuletzt genannten Merkmale (bessere H₂-Löslichkeit, niedrigere Temperatur) hätten den Fachmann jedoch bestenfalls eine Verbesserung des Hydroisomerisierungsergebnisses um etwa 0,5 %-Punkte in Richtung des thermodynamischen Gleichgewichts erwarten lassen. In überraschender Weise werden jedoch im erfindungsgemäßen Verfahren Verbesserungen um 5 bis 7 %-Punkte in Richtung auf das thermodynamische Gleichgewicht erzielt.

Die Erfindung betrifft ein Verfahren zur Herstellung von C₄-C₆-Alkenen mit innenständiger Doppelbindung durch Hydroisomerisierung von C₄-C₆-Alkenen mit endständiger Doppelbindung in Gegenwart von Wasserstoff an einem Katalysator mit einem Gehalt an einem Edelmetall der Gruppe VIII des Periodensystems der Elemente (Mendelejew), das gekennzeichnet ist durch die Verfahrensschritte
a) Einführen eines Feed aus temperierten C₄-C₆-Alkenen mit endständiger Doppelbindung, Wasserstoff und einem Rückführstrom mit einer Temperatur von 15 bis 60°C in einen Reaktor, der mit dem Edelmetall-haltigen Katalysator gefüllt ist,
b) Isomerisieren der im Feed enthaltenen C₄-C₆-Alkene mit endständiger Doppelbindung am Katalysator im Reaktor bei einer LHSV von 1 bis 20 h⁻¹, 15 bis 120°C und einem Druck, bei dem die C₄-C₆-Alkene mit endständiger Doppelbindung und der Rückführstrom in der flüssigen Phase vorliegen, zur weitgehenden Einstellung des thermodynamischen Gleichgewichts zwischen C₄-C₆-Alkenen mit endständiger Doppelbindung und C₄-C₆-Alkenen mit innenständiger Doppelbindung,
c) Aufteilung des vom Reaktor ablaufenden Produktstroms in einen Aufarbeitungsstrom und einen Rückführstrom, der im stationären Zustand das 0,1- bis 10-fache des Aufarbeitungsstroms beträgt,
d) Rückführung des Rückführstroms in Schritt a) und
e) Isolierung der C₄-C₆-Alkene mit innenständiger Doppelbindung aus dem Aufarbeitungsstrom.

Zwischen C₄-C₆-Alkenen mit endständiger Doppelbindung und C₄-C₆-Alkenen mit innenständiger Doppelbindung besteht ein thermodynamisches Gleichgewicht, das bei hohen Temperaturen stark zur Seite der C₄-C₆-Alkene mit endständiger Doppelbindung und bei niedrigen Temperaturen zur Seite der C₄-C₆-Alkene mit innenständiger Doppelbindung verschoben ist. Im Effluent eines Crackers (Steam-Cracker oder katalytischer Cracker) befindet sich daher stets ein hoher Anteil an C₄-C₆-Alkenen mit endständiger Doppelbindung, beispielsweise an Buten-1, verglichen mit dem Anteil an C₄-C₆-Alkenen mit innenständiger Doppelbindung, beispielsweise an Buten-2, der durch das Quenchen dieses Effluents eingefroren wird und nicht dem tatsächlichen Bedarf an den beiden jeweiligen Isomeren entspricht.

Das erfindungsgemäße Verfahren ermöglicht somit die Herstellung von Buten-2 aus Buten-1, von Penten-2 aus Penten-1 und eines Gemisches aus Hexen-2 und Hexen-3 aus Hexen-1; besondere Bedeutung kommt hierbei der Herstellung von Buten-2 aus Buten-1 zu, die im folgenden beispielhaft beschrieben wird. Der Fachmann kann diese Beschreibung auf die analoge Herstellung von Penten-2 bzw. Hexen-2/Hexen-3 in einfacher Weise übertragen.

Im erfindungsgemäßen Verfahren können die C₄-C₆-Alkene mit endständiger Doppelbindung in Form eines Kohlenwasserstoffstroms eingesetzt werden, der neben den C₄-C₆-Alkene mit endständiger Doppelbindung weitere gesättigte, einfach ungesättigte und zweifach ungesättigte Kohlenwasserstoffe mit 1-8 C-Atomen enthält.

So kann beispielsweise das Buten-1 als solches oder als Gemisch mit anderen Kohlenwasserstoffen eingesetzt werden. Besonders wichtig ist der Einsatz von Buten-1 in Form der technisch verfügbaren Kohlenwasserstoffgemische. Solche Gemische enthalten neben dem Buten-1 weitere gesättigte, einfach ungesättigte und zweifach ungesättigte Kohlenwasserstoffe sowie gegebenenfalls geringe Mengen acetylenisch ungesättigter Kohlenwasserstoffe mit beispielsweise 1 bis 6 C-Atomen, bei den C₅-/C₆-Alkenen auch bis zu 8 C-Atomen. Gemische dieser Breite im C-Zahlenbereich können bei einer rohen Destillation eines Cracker-Effluents gewonnen und erfindungsgemäß eingesetzt werden. Interessanter als ein solcher roher Destillationsschnitt ist jedoch der Einsatz eines feineren Destillationsschnittes, der beispielsweise im wesentlichen C₄-Kohlenwasserstoffe enthält, etwa neben dem Buten-1 n-Butan, i-Butan, cis-Buten-2, trans-Buten-2, i-Buten, Butadien, Vinylacetylen und untergeordnete Mengen Kohlenwasserstoffe der angrenzenden C₃- und C₅-Bereiche. In besonders bevorzugter Form wird statt eines solchen rohen C₄-Destillationsschnittes ein sogenanntes Raffinat 1 eingesetzt, welches durch geeignete Maßnahmen (beispielsweise durch Extraktion) weitgehend vom Butadien befreit worden ist. Typische Zusammensetzung eines Raffinats 1 aus einem Steam-Cracker sind:

| **Raff. 1-Komponente** | | |
|---|---|---|
| n-Butan | % | 10-20 |
| i-Butan | % | 0,5-1 |
| Buten-1 | % | 20-25 |
| Buten-2 (cis) | % | 5-8 |
| Buten-2 (trans) | % | 8-11 |
| i-Buten | % | 38-45 |
| Butadien | % | 0,1-0,3 |
| C₃-Kohlenwasserstoff | % | 0-1 |
| C₅-Kohlenwasserstoff | % | 0-1 |

Alternativ hierzu kann auch ein sogenanntes Raffinat 2 eingesetzt werden, das durch katalytische Umsetzung und dadurch Entfernung des im Raffinat 1 enthaltenen i-Butens erhalten wird. Allgemein können selbstverständlich die entsprechenden Stoffströme mit Gehalten an Penten-1 und/oder Hexen-1 eingesetzt werden. Schließlich ist es auch möglich, einen Stoffstrom einzusetzen, der Buten-1 und Penten-1 oder alle C₄-C₆-Alkene mit endständiger Doppelbindung enthält.

Wasserstoff kann im erfindungsgemäßen Verfahren in reiner oder technischer Form eingesetzt werden. Wirtschaftlich vorteilhaft kann beispielsweise ein in petrochemischen Anlagen anfallender, mit Methan und/oder Stickstoff vergesellschafteter Wasserstoff oder ein H₂-haltiges Restgas von petrochemischen Anlagen eingesetzt werden. Der H₂-Gehalt in solchen technischen oder reinen Wasserstoffen beträgt 70 bis 100 Vol.-% H₂, in Restgasen vielfach etwa 80 bis 90 Vol.-% H₂. Der Wasserstoff ist seine Menge grundsätzlich beliebig, beispielsweise 0,5 bis 20 Normliter (N1) pro 1 Buten-1, bevorzugt 1 bis 15 Nl, besonders bevorzugt 1 bis 5 Nl pro 1 Buten-1. Im oberen Teil des genannten Bereiches wird man jedoch mit einer merklichen Hydrierung des für Hydrierungen besonders anfälligen Buten-1, also mit der endständigen Doppelbindung (allgemein C₄-C₆-Alken-1), rechnen müssen. Im unteren Teil des genannten Bereiches wird man mit geringeren Reaktionsgeschwindigkeiten rechnen müssen, insbesondere wenn im Bereich tiefer Temperaturen und im Bereich höherer LHSV(Liquid Hourly Space Velocity)-Werte gearbeitet werden soll. Es ist daher erfindungsgemäß besonders vorteilhaft, wenn infolge der Vergrößerung des Massenstroms durch den Rückführstrom im Bereich niedriger H₂-Mengen gearbeitet werden kann. Ein weiterer Vorteil des erfindungsgemäßen Verfahrens ist der, daß das hydrieranfällige Butadien (allgemein C₄-C₆-Alkadien), sofern es im Feed vorhanden ist, bis unterhalb der Nachweisbarkeitsgrenze zu Buten-1 (allgemein C₄-C₆-Alken-1) hydriert wird, welches im Sinne der erfindungsgemäßen Hydroisomerisierung in Buten-2 (allgemein C₄-C₆-Alken mit innenständiger Doppelbindung) umgewandelt werden kann. Durch die gewünschte Hydrierung von beispielsweise Butadien und infolge einer nicht immer vollständig zu umgehenden Hydrierung von Buten-1 tritt jedoch neben der reinen katalytischen Wirkung des H₂ ein gewisser Verbrauch an H₂ auf, der ersetzt werden muß. Ansonsten kann der bei der Aufarbeitung anfallende H₂ wieder recyclisiert werden.

Als Edelmetalle der Gruppe VIII des Periodensystems der Elemente (Mendelejew), die eine Hydrieraktivität aufweisen und erfindungsgemäß eingesetzt werden können, seien Ru, Rh, Pd, Ir und Pt genannt. Von diesen sind Pd und Pt, insbesondere jedoch Pd bevorzugt. Die Edelmetalle werden stets in einer dispergierten Form auf einem Träger eingesetzt. Als Träger kommen beispielsweise anorganische Materialien, wie Al₂O₃, SiO₂ oder A-Kohle sowie weitere, dem Fachmann für diese Zwecke bekannte Träger in Betracht. Die Menge des Edelmetalls auf solchen anorganischen Trägern beträgt typischerweise 0,001 bis 10 Gew.-%, bevorzugt 0,1 bis 5 Gew.-%, bezogen auf das Gesamtgewicht des Trägers mit Edelmetall. Träger können beispielsweise aber auch solche organischer Natur sein, wie etwa Kationenaustauscher auf der Basis von Phenol-Formaldehydharzen mit eingeführten Sulfonsäuregruppen oder von Styrol-Divinylbenzol-Harzen, ebenfalls mit eingeführten Sulfonsäuregruppen.

Zur Durchführung des erfindungsgemäßen Verfahrens wird ein Reaktor benutzt, der mit dem oben beschriebenen Katalysator, der ein Edelmetall der Gruppe VIII des Periodensystems der Elemente enthält, gefüllt ist. Dieser Reaktor kann beispielsweise als Schachtreaktor, als Rohrreaktor, als Rohrbündelreaktor oder in einer anderen geeigneten Weise ausgebildet sein.

In diesen Reaktor wird ein Feed eingeführt, das beispielsweise aus Buten-1, Wasserstoff und dem bereits genannten Rückführstrom besteht. Das Buten-1 wird hierbei als solches oder in Form eines der oben beschriebenen Kohlenwasserstoffgemische eingesetzt. Das Buten-1 als solches oder in Form eines der obengenannten Kohlenwasserstoffgemische wird vor dem Einführen in den Reaktor auf eine Temperatur von 15 bis 60°C, bevorzugt 15 bis 40°C, besonders bevorzugt 15 bis 30°C, gebracht. In gleicher Weise wird der ebenfalls zum Feed gehörende Wasserstoff temperiert. Das Temperieren des H₂ kann hierbei gemeinsam mit dem Buten-1 bzw. einem das Buten-1 enthaltende Kohlenwasserstoffstrom geschehen. Ein separates Temperieren des H₂ kann unterbleiben, wenn der H₂ bereits eine geeignete Temperatur besitzt. In gleicher Weise wird der Rückführstrom temperiert, wobei ebenfalls vor dem Temperieren ein Vermischen mit dem Buten-1 bzw. einem das Buten-1 enthaltenden Kohlenwasserstoffstrom und/oder dem H₂ vorgenommen werden kann. Ein separates Temperieren des Rückführstroms kann, wie dies bereits für H₂ ausgeführt wurde, unterbleiben, wenn der Rückführstrom bereits eine geeignete Temperatur hat. Grundsätzlich können die Bestandteile des einzuführenden Feeds getrennt an verschiedenen Stellen des Reaktors eingeführt werden. Es ist jedoch günstig, die Bestandteile des Feeds vorher zu mischen und dann in den Reaktor einzuführen.

Das in den Reaktor eingeführte Feed aus beispielsweise Buten-1, H₂ und dem Rückführstrom wird im Reaktor bei 15 bis 120°C, bevorzugt 20 bis 80°C, besonders bevorzugt 20 bis 60°C, hydroisomerisiert. Das Reaktionsgemisch liegt hierbei mit Ausnahme von nicht völlig gelösten Anteilen an H₂ in der flüssigen Phase vor. Das Reaktionsgemisch wird mit einer LHSV (Liquid Hourly Space Velocity) von 1 bis 20 h⁻¹ (Liter flüssiges Reaktionsgemisch pro Liter Katalysator pro Stunde), bevorzugt 3 bis 15 h⁻¹, besonders bevorzugt 5 bis 12 h⁻¹ über den Katalystor geleitet. Zur Aufrechterhaltung der flüssigen Phase, wie oben beschrieben, wird im Reaktor ein geeigneter Druck aufrecht erhalten. Dieser Druck ist in allgemeiner Form der autogene Druck, der sich automatisch einstellt. Beispielsweise sei ein Bereich von 5 bis 30 bar, bevorzugt 8 bis 20 bar, genannt. Unter diesen Reaktionsbedingungen stellt sich das thermodynamische Gleichgewicht beispielsweise zwischen Buten-1 und Buten-2 ein. In der oben erwähnten Weise liegt dieses thermodynamische Gleichgewicht bei tieferer Temperatur stärker auf der Weise des gewünschten Buten-2; es ist daher wünschenswert und erfindungsgemäß möglich, eine möglichst tiefe Reaktionstemperatur einzustellen, wobei jedoch eine ausreichende Hydroisomerisierungsgeschwindigkeit gewährleistet bleiben muß.

Der vom Reaktor ablaufende Produktstrom wird in einen Aufarbeitungsstrom und einen Rückführstrom aufgeteilt. Der Rückführstrom beträgt hierbei das 0,1 bis 10-fache des Aufarbeitungsstroms, bevorzugt das 0,1- bis 4-fache, besonders bevorzugt das 0,1- bis 2-fache des Aufarbeitungsstroms. Der Rückführstrom wird an den Eingang des Reaktors zurückgeführt und dort im Verfahrensschritt a) gemeinsam oder separat beispielsweise mit dem Buten-1 und dem Wasserstoff als Feed in den Reaktor eingeführt. Der Rückführstrom kann hierzu, wie ebenfalls weiter oben bereits ausgeführt wurde, separat oder gemeinsam mit Buten-1 und/oder Wasserstoff temperiert werden. Es kann nützlich sein, den vom Reaktor ablaufenden Produktstrom vor der Aufteilung in einen Aufarbeitungsstrom und einen Rückführstrom in einem Wärmeaustauscher auf eine geeignete Temperatur zu bringen. Es kann jedoch gleichermaßen nützlich sein, den vom Reaktor ablaufenden Produktstrom erst nach seiner Aufteilung auf eine geeignete Temperatur zu bringen; hierbei hat gegebenenfalls der Rückführstrom bereits eine zur Einführung in den Reaktor geeignete Temperatur, so daß er nicht mehr separat temperiert zu werden braucht. In analoger Weise kann auch der Aufarbeitungsstrom erst nach der Aufteilung des vom Reaktor ablaufenden Produktstroms in einem Wärmeaustauscher auf eine geeignete Temperatur gebracht werden, die für die nachfolgende Aufarbeitung geeignet ist. Die Aufarbeitung des Produktstroms, der nunmehr weitgehend an das thermodynamische Gleichgewicht beispielsweise zwischen Buten-2 und Buten-1 angenähert ist, erfolgt nach fachmännisch bekannten Methoden, beispielsweise durch Destillation oder Oligomerisierung.

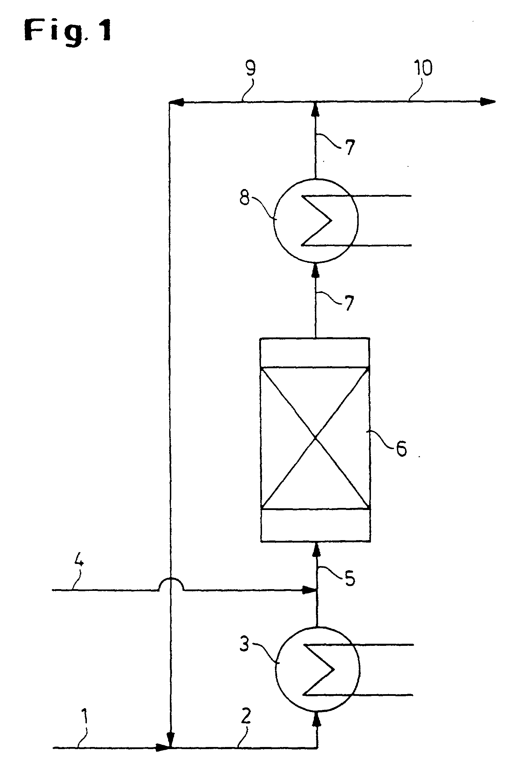

Eine der möglichen Durchführungsformen des erfindungsgemäßen Verfahrens, sei anhand der beiliegenden Fig. 1 dargestellt und am Beispiel der Umwandlung von Buten-1 in Buten-2 beschrieben. In Fig. 1 bedeuten (1) Buten-1 oder einen das Buten-1 enthaltenden Kohlenwasserstoffstrom, der sich noch nicht im thermodynamischen Gleichgewicht zwischen Buten-2 und Buten-1 befindet; der Strom (1) wird in einer erfindungsgemäß möglichen und in Fig. 1 dargestellten Ausführungsform mit einem Rückführstrom (9) vereinigt und als vereinigter Strom (2) über einen Wärmeaustauscher (3) auf die gewünschte Temperatur eingestellt; nach der Temperierung wird beispielsweise diesem vereinigten Strom über die Leitung (4) Wasserstoff in der vorgesehenen Menge zugefügt und als vereinigtes Feed über die Leitung (5) in den Reaktor (6) eingeführt; der in (6) hydroisomerisierte Produktstrom läuft über die Leitung (7) beispielsweise über einen Wärmeaustauscher (8) und wird danach in den Aufarbeitungsstrom (10) und den Rückführstrom (9), der dem Buten-1 zugemischt wird, aufgeteilt.

### Ausführungsbeispiele

### Beispiele 1-6

In einer Apparatur wie in Fig. 1 wurde ein Buten-1 haltiges Feed unter den in der folgenden Tabelle angegebenen Bedingungen umgesetzt.

| Beispiel-Nr. | 1 | 2 | 3 | 4 | 5 | 6 |
|---|---|---|---|---|---|---|
| Druck (bar) | 17 | 14 | 14 | 14 | 14 | 14 |
| H2 (Nl/ l Gesamt-Feed) | 7 | 4 | 3 | 8 | 5 | 6 |
| LHSV (Gesamt) | 8 | 9 | 9 | 8 | 8 | 8 |
| Kreislaufanteil (%) | 0 | 15 | 30 | 17 | 17 | 0 |
| Einsatztemperatur (°C) | 20 | 19 | 19 | 16 | 16 | 16 |
| Ausgangstemperatur (°C) | 66 | 51 | 46 | 68 | 49 | 52 |
| Einsatz-Buten-1 (Gew.-%) | 23,6 | 20,9 | 17,9 | 21,4 | 21,4 | 25,2 |
| Ausgangs-Buten-1 (Gew.-%) | 8,1 | 3,5 | 2,2 | 4 | 4,5 | 9 |
| Hydrierung von Butenen (%) | 4,5 | 2,3 | 1,7 | 5,3 | 3,2 | 3,6 |
| Hydrierung von Butadien (%) | 100 | 100 | 100 | 100 | 100 | 100 |

Erläuterungen zur Tabelle:
Vergleichsbeispiele 1 und 6: ohne Kreislauffahrweise (24-25% Buten-1)
Beispiele 2,4 und 5: mit 15 bis 17 % Kreislaufanteil (Buten-1-Verdünnung)
Beispiel 3: mit 30 % Kreislaufanteil (Buten-1-Verdünnung)

Vergleich des Vergleichsbeispiels 1 mit den Beispielen 2 und 3 (Vorteile bei Erhöhung des Kreislaufanteils):
- Verbesserung der Isomerisierung (geringe Buten-1-Gehalte im Ausgang)
- Verwendung geringerer H2-Mengen durch Minimierung der Nebenreaktionen (hier: Hydrierung der Butene)
- Geringere Temperaturerhöhung im Reaktor

Vergleich der Beispiele 2, 4 und 5 (Vorteile bei Verwendung geringerer H2-Mengen bei konstantem Kreislaufanteil):
- Niedrigere Temperaturen am Reaktorausgang
- Verbesserung der Isomerisierung bei niedrigen Temperaturen durch günstigere Lage zum thermodynamischen Gleichgewicht
- Geringere Verluste an Butenen durch Hydrierung

Vergleich der Vergleichsbeispiele 1 und 6:
Ohne Kreislauffahrweise erfolgt selbst bei günstigen sonstigen Bedingungen eine noch nicht ausreichende Isomerisierung.

## Patentansprüche

1. Verfahren zur Herstellung von C₄-C₆-Alkenen mit innenständiger Doppelbindung durch Hydroisomerisierung von C₄-C₆-Alkenen mit endständiger Doppelbindung in Gegenwart von Wasserstoff an einem Katalysator mit einem Gehalt an einem Edelmetall der Gruppe VIII des Periodensystems der Elemente (Mendelejew), **gekennzeichnet durch** die Verfahrensschritte
a) Einführen eines Feed aus temperierten C₄-C₆-Alkenen mit endständiger Doppelbindung, H₂ und einem Rückführstrom mit einer Temperatur von 15 bis 60°C in einen Reaktor, der mit dem Edelmetall-haltigen Katalysator gefüllt ist,
b) Isomerisieren der im Feed enthaltenen C₄-C₆-Alkene mit endständiger Doppelbindung am Katalysator im Reaktor bei einer LHSV von 1 bis 20 h⁻¹, 15 bis 120°C und einem Druck, bei dem die C₄-C₆-Alkene mit endständiger Doppelbindung und der Rückführstrom in der flüssigen Phase vorliegen, zur weitgehenden Einstellung des thermodynamischen Gleichgewichts zwischen C₄-C₆-Alkenen mit endständiger Doppelbindung und C₄-C₆-Alkenen mit innenständiger Doppelbindung,
c) Aufteilung des vom Reaktor ablaufenden Produktstroms in einen Aufarbeitungsstrom und einen Rückführstrom, der im stationären Zustand das 0,1- bis 10-fache des Aufarbeitungsstroms beträgt,
d) Rückführung des Rückführstroms in Schritt a) und
e) Isolierung der C₄-C₆-Alkene mit innenständiger Doppelbindung aus dem Aufarbeitungsstrom.

2. Verfahren nach Anspruch 1, **dadurch gekennzeichnet, daß** die C₄-C₆-Alkene mit endständiger Doppelbindung in Form eines Kohlenwasserstoffstroms eingesetzt werden, der neben C₄-C₆-Alkenen mit endständiger Doppelbindung weitere gesättigte, einfach ungesättigte und zweifach ungesättigte Kohlenwasserstoffe mit 1 bis 8 C-Atomen enthält.

3. Verfahren nach Ansprüchen 1 und 2, **dadurch gekennzeichnet, daß** die H₂-Menge 0,5 bis 20 Nl/l Buten-1, bevorzugt 1 bis 15 Nl/l Buten-1, besonders bevorzugt 1 bis 5 Nl/l Buten-1 beträgt.

4. Verfahren nach Ansprüchen 1 bis 3, **dadurch gekennzeichnet, daß** die Vorwärmtemperatur 15 bis 40°C, bevorzugt 15 bis 30°C, und die Reaktionstemperatur 20 bis 80°C, bevorzugt 20 bis 60°C beträgt.

5. Verfahren nach Ansprüchen 1 bis 4, **dadurch gekennzeichnet, daß** der Druck im Reaktor 5 bis 30 bar, bevorzugt 8 bis 20 bar, beträgt.

6. Verfahren nach Ansprüchen 1 bis 5, **dadurch gekennzeichnet, daß** die LHSV 3 bis 15 h⁻¹, bevorzugt 5 bis 12 h⁻¹, beträgt.

7. Verfahren nach Ansprüchen 1 bis 6, **dadurch gekennzeichnet, daß** der Rückführstrom das 0,1- bis 4-fache, bevorzugt das 0,1- bis 2-fache, des Aufarbeitungsstroms beträgt.

8. Verfahren nach Ansprüchen 1 bis 7, **dadurch gekennzeichnet, daß** als Edelmetall Palladium oder Platin, bevorzugt Palladium eingesetzt wird.

9. Verfahren nach Ansprüchen 1 bis 8, **dadurch gekennzeichnet, daß** sich das Edelmetall auf Al₂O₃ als Träger befindet.

10. Verfahren nach Ansprüchen 1 bis 9, **dadurch gekennzeichnet, daß** es auf die Hydroisomerisierung von Buten-1 zu Buten-2 ausgerichtet ist.

11. Verfahren nach Anspruch 10, **dadurch gekennzeichnet, daß** Buten-1 in Form eines Kohlenwasserstoffstroms eingesetzt wird, der einen C₄-Destillationsschnitt darstellt, der neben Buten-1 weitere Kohlenwasserstoffe aus der Gruppe n-Butan, i-Butan, cis-Buten-2, trans-Buten-2, i-Buten, Butadien und untergeordnete Mengen Kohlenwasserstoffe der angrenzenden C₃- und C₅-Bereiche enthält, und bevorzugt in Form eines weitgehend von Butadien-befreiten C₄-Destillationsschnittes eingesetzt wird.

## Claims

1. Process for preparing C₄-C₆-alkenes having an internal double bond by hydroisomerization of C₄-C₆-alkenes having a terminal double bond in the presence of hydrogen on a catalyst having a content of a noble metal of group VIII of the Periodic Table of the Elements (Mendeleev), **characterized by** the process steps
a) introducing a feed of heated C₄-C₆-alkenes having a terminal double bond, H₂ and a recycle stream having a temperature of 15 to 60°C into a reactor which is charged with the noble-metal containing catalyst,
b) isomerizing the C₄-C₆-alkenes having a terminal double bond present in the feed at the catalyst in the reactor at an LHSV of 1 to 20 h⁻¹, 15 to 120°C and a pressure at which the C₄-C₆-alkenes having a terminal double bond and the recycle stream are present in the liquid phase, for substantial establishment of the thermodynamic equilibrium between C₄-C₆-alkenes having a terminal double bond and C₄-C₆-alkenes having an internal double bond,
c) dividing the product stream exiting from the reactor into a work-up stream and a recycle stream which in the steady state is 0.1 to 10 times the work-up stream,
d) recycling the recycle stream to step a) and
e) isolating the C₄-C₆-alkenes having an internal double bond from the work-up stream.

2. Process according to Claim 1, **characterized in that** the C₄-C₆-alkenes having a terminal double bond are used in the form of a hydrocarbon stream which, in addition to C₄-C₆-alkenes having a terminal double bond, comprises further saturated, monounsaturated and diunsaturated hydrocarbons having 1 to 8 carbon atoms.

3. Process according to Claims 1 and 2, **characterized in that** the H₂ rate is 0.5 to 20 1 S.T.P./l of 1-butene, preferably 1 to 15 1 S.T.P./l of 1-butene, particularly preferably 1 to 5 1 S.T.P./l of 1-butene.

4. Process according to Claims 1 to 3, **characterized in that** the preheating temperature is 15 to 40°C, preferably 15 to 30°C, and the reaction temperature is 20 to 80°C, preferably 20 to 60°C.

5. Process according to Claims 1 to 4, **characterized in that** the pressure in the reactor is 5 to 30 bar, preferably 8 to 20 bar.

6. Process according to Claims 1 to 5, **characterized in that** the LHSV is 3 to 15 h⁻¹, preferably 5 to 12 h⁻¹.

7. Process according to Claims 1 to 6, **characterized in that** the recycle stream is 0.1 to 4 times, preferably 0.1 to 2 times, the work-up stream.

8. Process according to Claims 1 to 7, **characterized in that** the noble metal used is palladium or platinum, preferably palladium.

9. Process according to Claims 1 to 8, **characterized in that** the noble metal is present on Al₂O₃ as support.

10. Process according to Claims 1 to 9, **characterized in that** it is directed towards the hydroisomerization of 1-butene to give 2-butene.

11. Process according to Claim 10, **characterized in that** 1-butene is used in the form of a hydrocarbon stream which represents a C₄-distillation fraction which, in addition to 1-butene, comprises other hydrocarbons selected from the group consisting of n-butane, i-butane, cis-2-butene, trans-2-butene, i-butene, butadiene and minor amounts of hydrocarbons of the adjoining C₃- and C₅- ranges, and is preferably used in the form of a C₄-distillation fraction substantially freed from butadiene.

## Revendications

1. Procédé de production d'alcènes en C₄-C₆ à double liaison interne par hydroisomérisation d'alcènes en C₄-C₆ à double liaison terminale en présence d'hydrogène sur un catalyseur ayant une teneur en un métal noble du groupe VIII du système périodique des éléments (Mendeleiev), **caractérisé par** les étapes de procédé
a) introduction d'une alimentation constituée par des alcènes en C₄-C₆ à double liaison terminale maintenus à température constante, H₂ et un courant de recyclage d'une température de 15 à 60°C dans un réacteur qui est rempli de catalyseur contenant un métal noble,
b) isomérisation des alcènes en C₄-C₆ à double liaison terminale contenus dans l'alimentation sur le catalyseur dans le réacteur, à une LHSV de 1 à 20 h⁻¹, à 15 à 120°C et à une pression à laquelle les alcènes en C₄-C₆ à double liaison terminale et le courant de recyclage sont en phase liquide, pour établir sensiblement l'équilibre thermodynamique entre les alcènes en C₄-C₆ à double liaison terminale et les alcènes en C₄-C₆ à double liaison interne,
c) division du courant de produit s'écoulant du réacteur en un courant de traitement et un courant de recyclage qui, à l'état stationnaire, représente 0,1 à 10 fois le courant de traitement,
d) recyclage du courant de recyclage dans l'étape a) et
e) isolement des alcènes en C₄-C₆ à double liaison interne à partir du courant de traitement.

2. Procédé selon la revendication 1, **caractérisé en ce que** les alcènes en C₄-C₆ à double liaison terminale sont utilisés sous forme d'un courant d'hydrocarbures qui, outre des alcènes en C₄-C₆ à double liaison terminale, contient d'autres hydrocarbures ayant 1 à 8 atomes de C saturés, monoinsaturés et à deux insaturations.

3. Procédé selon les revendications 1 et 2, **caractérisé en ce que** la quantité de H₂ est de 0,5 à 20 Nl/l de but-1-ène, de préférence de 1 à 15 Nl/l de but-1-ène, de préférence encore de 1 à 5 Nl/l de but-1-ène.

4. Procédé selon les revendications 1 à 3, **caractérisé en ce que** la température de préchauffage est de 15 à 40°C, de préférence de 15 à 30°C, et la température de réaction est de 20 à 80°C, de préférence de 20 à 60°C.

5. Procédé selon les revendications 1 à 4, **caractérisé en ce que** la pression dans le réacteur est de 5 à 30 bar, de préférence de 8 à 20 bar.

6. Procédé selon les revendications 1 à 5, **caractérisé en ce que** la LHSV est de 3 à 15 h⁻¹, de préférence de 5 à 12 h⁻¹.

7. Procédé selon les revendications 1 à 6, **caractérisé en ce que** le courant de recyclage représente 0,1 à 4 fois, de préférence 0,1 à 2 fois le courant de traitement.

8. Procédé selon les revendications 1 à 7, **caractérisé en ce que** le palladium ou le platine, de préférence le palladium, est utilisé comme métal noble.

9. Procédé selon les revendications 1 à 8, **caractérisé en ce que** le métal noble est situé sur Al₂O₃ comme support.

10. Procédé selon les revendications 1 à 9, **caractérisé en ce qu'**il est destiné à l'hydroisomérisation du but-1-ène en but-2-ène.

11. Procédé selon la revendication 10, **caractérisé en ce que** le but-1-ène est utilisé sous forme d'un courant d'hydrocarbures qui constitue une coupe de distillation en C₄ qui, outre du but-1-ène, contient d'autres hydrocarbures du groupe du n-butane, du i-butane, du cis-but-2-ène, du trans-but-2-ène, du i-butène, du butadiène et des quantités inférieures d'hydrocarbures des domaines en C₃ et C₅ voisins, et est utilisé de préférence sous forme d'une coupe de distillation en C₄ sensiblement débarrassée du butadiène.
